# EUROPEAN PATENT APPLICATION

(11) **EP 4 209 237 A1**
(43) Date of publication of application: **12.07.2023**
(21) Application number: 22305010.5
(22) Date of filing: 07.01.2022
(51) Int. Cl.: A61M 5/31, A61M 5/28, A61M 5/315, A61M 5/178

(54) **SPECIFIC BARREL FOR AN INJECTION DEVICE, ASSEMBLY AND INJECTION DEVICE WITH SUCH A BARREL**

(71) Applicant: Becton, Dickinson and Company, Franklin Lakes, NJ 07417-1880 (US); Becton Dickinson France, 38800 Le Pont-de-Claix (FR)
(72) Inventor: EUVRARD, Nicolas, Plainsboro NJ 08536 (US); GARREC, Ronan, 38640 Claix (FR); VAXELAIRE, Jérémie, 38000 Grenoble (FR)
(74) Representative: Germain Maureau

(57) **Abstract**

The present invention relates to a barrel (1) for an injection device (20), said barrel (1) having an inner surface (4) provided with at least one radially inwardly directed longitudinal rib (6) extending longitudinally on a predetermined distance D, said longitudinal rib (6) dividing the interior (5) of said barrel (1) into a transitional chamber (7), a proximal chamber (8) and optionally a distal chamber (9). The invention further relates to an assembly comprising such a barrel (1) and at least two stoppers. The invention also relates to an injection device comprising such an assembly, a first component stored either in the transitional chamber (7) or in the distal chamber (9) and a second component stored in the proximal chamber (8).

## Description

The present invention relates to a barrel for an injection device, the inner wall of said barrel comprising a specific rib allowing, at the time of the injection step, the mixing of two components initially stored separately within said barrel. The present invention further relates to an assembly comprising such a barrel and at least two stoppers, and to an injection device comprising such an assembly.

In this application, the distal end of a component or of a device means the end furthest away from the hand of the user and the proximal end means the end closest to the hand of the user, when the component or device is in the use position. Similarly, in this application, the terms "in the distal direction" and "distally" mean in the direction of the injection, and the terms "in the proximal direction" and "proximally" mean in the direction opposite to the direction of injection.

Injection devices typically include an elongate container such as a barrel having opposed proximal and distal ends with a chamber therebetween for receiving a product, such as a drug. The distal end of the barrel tapers into a channel forming a passageway communicating with the chamber. The passageway is intended to be connected to a piercing element, such as a needle cannula or a needle hub, for the delivery of the product stored in the chamber. The proximal end of the barrel is open and intended to be closed by a stopper, to which a plunger rod is connected. At the time of injection, a distal force applied on the plunger rod causes the stopper to be moved in the distal direction and to expel the product stored in the chamber through the passageway and then the needle cannula.

Depending on the nature or form of the drug contained in the chamber of the barrel, it may be desirable to deliver a number of discrete substances held within the chamber. These discrete substances may preferably be held separate from one another until time of delivery to the patient. It is necessary, then, that the chamber defined between the proximal and distal ends of the barrel be divided into a discrete number of chambers, each capable of holding a substance intended for delivery to a patient.

It is therefore desirable to construct an injection device capable of simultaneously storing a drug together with any components which are to be administered, with the ability to keep the drug and the components separate until administration is desired. Drugs storable in injections devices can be in liquid form or in dry form. It is often desirable to store drugs in dry form since, depending on the type of drug, it will display a longer shelf-life compared to the liquid form of the drug.

As is known in the art, one way to reduce a liquid drug to dry form is through a lyophilisation process. In this process, liquid drugs are subjected to a freeze-drying, or lyophilisation, process, that reduces the liquid drug to a dried powder or granular form. For purposes of simplicity, reference in the present application to a drug in lyophilized form is intended to encompass any dry drug, all of which require reconstitution under a liquid form by addition of a diluent for example, prior to delivery.

Alternatively, a liquid drug to be injected may be the result of the mixing of two initial liquid substances that need to be stored separately and mixed together just before the delivery to a patient. In other cases, two liquid substances may need to be stored separately, and then injected sequentially, one after the other.

In order to deliver either dry drugs that need to be reconstituted with a diluent prior to injection, or liquid drugs that are the result of the mixing of two liquid components, dual chamber injection devices with an external by-pass have been provided, as described for example in US 5, 865, 798. Such injection devices comprise a barrel having an external protruding by-pass. As a result, the outer diameter of such barrels increases on a certain length of the barrel, resulting in an asymmetrical injection device. This has the drawback that the devices cannot be stored and conveyed in nests originally designed for conventional injection devices that are free of external by-passes. With barrels having an external by-pass, care must be taken to orientate the injection devices along a specific direction during conveying operations, during filling and finishing operations. In addition, additional care must also be taken during label fixation step, as the external by-pass prevents from securely affixing a label to the outside surface of the barrel. Specific blisters may also be designed and manufactured for the packaging of these injection devices.

In addition, the footprint of a dual chamber barrel with an external by-pass is higher than that of a conventional barrel. As a consequence, when dual chamber barrels with external by-passes are used for storing lyophilized drugs, the number of barrels than can be placed in the lyophilisation chamber during the lyophilisation step is significantly reduced compared to the number of conventional barrels the lyophilisation chamber could theoretically receive.

Another drawback encountered with dual chamber barrels having an external by-pass is the complexity of their manufacturing process. Indeed, in the case of glass made dual chamber barrels, the forming process may require the insertion of a specific tool inside the barrel, where such tool is moved towards the inner wall of the barrel to create the by-pass, and where such tool needs then to be extracted from the inside of the barrel. Such a process therefore requires several steps necessitating a precautious handling of the barrel and of the tool. Risks of contaminating the interior of the barrel with the tool or of generating scratches and/or cracks when manipulating the tool inside the barrel are not excluded.

There is therefore the need for a barrel for an injection device, said barrel comprising at least two chambers for separately storing two components intended either to be mixed together before delivery or to be sequentially delivered, i.e. one after the other, where such barrel would be easy to manufacture and easy to use and manipulate. In particular, there is a need for such a barrel, where such barrel would help streamlining the various operations, such as filling, lyophilization, conveying, packaging, storing, such a barrel is intended to be submitted to during the time of manufacture and use of the injection device.

A first aspect of the invention is a barrel for an injection device, said barrel being formed of a tubular wall with an open proximal end and a distal end defining a passageway for a product to be stored in said barrel, said tubular wall having an inner surface defining an interior of said barrel, wherein said inner surface is provided with at least one radially inwardly directed longitudinal rib extending longitudinally on a predetermined distance D, said longitudinal rib being dimensioned and positioned to divide the interior of said barrel into at least two chambers, a transitional chamber, extending on said predetermined distance D, and a proximal chamber located proximally to said transitional chamber. In embodiments, the longitudinal rib is dimensioned and positioned to divide the interior of said barrel into three chambers which are said transitional chamber, said proximal chamber, and a distal chamber which is located distally to said transitional chamber.

A second aspect of the invention is an assembly comprising:
- At least one barrel as defined above, and
- At least one first stopper and one second stopper, each of said first and second stoppers being shaped and dimensioned to be slidable within the interior of the barrel, each of said first and second stoppers being provided with at least one outer annular rib dimensioned to sealingly contact the inner surface of said tubular wall when said stopper is located within said proximal chamber.

A third aspect of the invention is an injection device comprising a barrel or an assembly as defined above.

The barrel of the invention allows storing two components in two separate chambers: for example, a first component may be stored in the transitional chamber or in the distal chamber, and a second component may be stored in the proximal chamber. As will appear from the description below, the barrel of the invention shows a global external volume identical to that of a conventional barrel free of any by-pass. Actually, the barrel of the invention has no protruding external by-pass. The outer global shape and volume of the barrel of the invention allows storing the injection devices that are constructed with said barrel in nests designed for conventional injection devices. As a result, a large number of operations requested during the manufacture of the injection device, such as conveying, filling, finishing, label affixing, packaging, transporting and storing, are significantly simplified and streamlined thanks to the barrel of the invention compared to dual-chamber syringes with an external by-pass.

The barrel of the invention, thanks to its outer global volume similar to that of conventional barrels having no by-pass, shows a reduced footprint compared to that of barrels with an external by-pass. This allows lyophilizers to be able to operate at a higher capacity than when syringes having external by-passes are used.

The manufacture of the barrel of the invention is also greatly simplified compared to the manufacture of a barrel having an external by-pass. Indeed, as will appear from the description below, the manufacture of a glass made barrel of the invention does not necessitate that a tool be inserted within the barrel, thereby significantly reducing the risks that the barrel be contaminated or damaged.

In addition, the assembly of the invention allows either storing separately in the transitional chamber and in the proximal chamber two liquid components intended to be delivered sequentially or proceeding to the mixing of the two components stored in the proximal chamber and in the distal chamber of the barrel in order for example to reconstitute the liquid drug to be delivered, while using stoppers that may be chosen from simple conventional stoppers, such as stoppers generally used with conventional barrels having no by-pass. Indeed, as will appear more clearly from the description below, in the barrel of the invention, the radially inwardly directed longitudinal rib provided on the inner surface of the wall of the barrel will create a leak path when a stopper, such as a conventional stopper, is located within the transitional chamber. Thanks to this leak path, the component stored in the proximal chamber will be allowed to transition from the proximal chamber to the transitional chamber, and/or further to the distal chamber if the distal chamber is present.

In addition, as will appear from the description below, with the barrel and assembly of the invention, the dead volume, in which a part of the drug to be delivered may be trapped and definitely lost, is particularly reduced.

The barrel of the invention is formed of a tubular wall having a proximal end and a distal end. The tubular wall may be made of glass or of plastic material. The distal end of the tubular wall defines a passageway for the product, such as a liquid drug, to be delivered. The proximal end is open and is intended to be closed by a stopper once the barrel is filled with the components to be stored. The proximal end of the tubular wall may form an outer flange. Alternatively, the proximal end of the tubular wall may not form any outer flange and a separate removable outer flange may be provided at the proximal end of the barrel.

The tubular wall has an inner surface defining an interior of the barrel. The inner surface is provided with at least one radially inwardly directed longitudinal rib extending longitudinally on a predetermined distance D. The longitudinal rib is dimensioned and positioned to divide the interior of the barrel into at least a first and a second chambers, which are a transitional chamber, extending on the predetermined distance D, and a proximal chamber located proximally to the transitional chamber, and optionally into a third chamber, which is a distal chamber located distally to the transitional chamber. The longitudinal rib therefore extends parallel to a longitudinal axis of the tubular wall, on a predetermined distance D, said predetermined distance D constituting a limited portion of the global length of the tubular wall. When the barrel comprises two chambers only, namely the transitional chamber and the proximal chamber, then the longitudinal rib extends up to the distal end of the barrel. In other words, the distal end of the transitional chamber is common with the distal end of the tubular wall. When the barrel comprises three chambers, then the longitudinal rib is positioned between the proximal end and the distal end of the tubular wall. The transitional chamber therefore separates the distal chamber, the distal end of which communicates with the passageway, from the proximal chamber, the proximal end of which is common with the proximal end of the tubular wall.

The longitudinal rib is provided on the inner surface of the tubular wall and is directed radially inwardly, in other words towards a central longitudinal axis of the tubular wall. As a consequence, as mentioned above, no part of the longitudinal rib protrudes outwardly from the outer globally cylindrical surface of the barrel.

The barrel may comprise a plurality of such longitudinal ribs, which are preferably distributed along a circumference of the tubular wall. When a plurality of longitudinal ribs are present, all the longitudinal ribs are preferably confined within the transitional chamber. For example, all the longitudinal ribs may show the same length and extend along the predetermined distance D corresponding to the length of transitional chamber.

In embodiments, the longitudinal ribs are regularly distributed along a circumference of the tubular wall. Alternatively, the longitudinal ribs may be confined within a restricted arc of a circumference of the tubular wall.

In embodiments, the circumferential length of the longitudinal rib(s) may vary from a proximal end of the longitudinal rib(s) to a distal end of the longitudinal rib(s). For example, the circumferential length of a longitudinal rib may be smaller at the proximal end of the longitudinal rib than at its distal end. Such embodiments create a progressive increase, in the distal direction, of the width of the leak path created by the presence of the longitudinal rib, thereby generating a smooth increase of the gliding force needed to move the stopper distally in the transitional chamber.

In embodiments, the radial inward height of the longitudinal rib(s) may vary from a proximal end of the longitudinal rib(s) to a distal end of the longitudinal rib(s). For example, the radial inward height of a longitudinal rib may be smaller at the proximal end of the longitudinal rib than at its distal end. Such embodiments generate a progressive increase, in the distal direction, of the height of the leak path created by the presence of the longitudinal rib : this allows a smooth increase of the gliding force needed to move the stopper distally in the transitional chamber.

In embodiments, the radial inward height of a longitudinal rib gradually increases from 0 at the proximal end of the longitudinal rib to a significant value at the distal end of the longitudinal rib, said significant value being chosen to create a step in the inner surface of the tubular wall. The presence of such a step generates a clear drop in the gliding force needed to move the stopper distally in the transitional chamber, thereby informing the user that the stopper leaves the transitional chamber. Such a step also constitutes a visual indication for the user that the stopper exits the transitional chamber and enters the distal chamber.

In other embodiments, the radial inward height of a longitudinal rib gradually increases from 0 to a maximum value and then decreases back to 0 along the length of the longitudinal rib. Such an embodiment allows varying the effort the user needs to exert to move the stopper distally in the transitional chamber: indeed, in such an embodiment, the gliding force needed to move the stopper smoothly increases up to the maximum value of the height of the longitudinal rib and then smoothly decreases until the distal end of the longitudinal rib.

In embodiments, the circumferential length of a longitudinal rib is smaller at the proximal end of the longitudinal rib than at its distal end, and the radial inward height of the longitudinal rib gradually increases from 0 at the proximal end of the longitudinal rib to a significant value to form a step at the distal end of the longitudinal rib. Such an embodiment combines the benefits described above. In particular, in such an embodiment, one obtains an even smoother increase of the gliding force needed to move the stopper distally in the transitional chamber.

The barrel of the invention may be made of plastic material and may in such a case be obtained by a moulding process. Alternatively, the barrel of the invention may be made of glass. In such a case, the formed barrel is rotated and heated and a groove is formed on the external surface of the barrel, either by means of a forming wheel having a pattern mirroring said groove, said wheel rotating above the barrel simultaneously with the barrel, or by a punching tool coming in contact with the barrel. The external groove thus created will form the longitudinal rib of the barrel. In both cases, no tool needs to be inserted inside the barrel to form the longitudinal rib. Therefore, risks of contamination of the interior of the barrel or of damaging the barrel are avoided.

The barrel of the invention is intended to be used with two stoppers in the manner which will now be described. For example, in a before use or storage configuration of an injection device comprising a barrel of the invention provided with two chambers only, a first component may be stored in the transitional chamber, a first stopper may be located at the distal end of the proximal chamber, a second component may be stored in the proximal chamber, and a second stopper may close the proximal end of the proximal chamber. Alternatively, in a before use configuration, or storage configuration, of an injection device comprising a barrel of the invention provided with three chambers, a first component may be stored in the distal chamber, a first stopper may be located at the distal end of the proximal chamber, a second component may be stored in the proximal chamber, and a second stopper may close the proximal end of the proximal chamber. In both examples, the first stopper therefore separates the first component from the second component.

The first and the second stoppers are shaped and dimensioned to be slidable within the interior of the barrel, i. e. within the proximal chamber, the transitional chamber and the distal chamber when present. Each stopper is provided with at least one outer annular rib dimensioned to sealingly contact the inner surface of the tubular wall when the stopper is located within the proximal chamber. Usually, the inner diameter of the distal chamber, when present, will be identical to that of the proximal chamber. As a result, an outer annular rib dimensioned to sealingly contact the inner surface of the tubular wall when the stopper is located within the proximal chamber will automatically be also dimensioned to sealingly contact the inner surface of the tubular wall when the stopper is located within the distal chamber. By "sealingly contacting" the inner surface of the tubular wall, is meant in the present application that, in the proximal chamber, and in the distal chamber when present, the at least one outer annular rib of the stopper contacts the inner surface of the tubular wall on its entire circumference and realizes a leak tight seal preventing all passage of any fluid or dry form of a component from one side of the outer annular rib to the other side. In embodiments, each stopper is provided with a plurality, for example three, outer annular ribs dimensioned to sealingly contact the inner surface of said tubular wall when said stopper is located within said proximal chamber, and within said distal chamber when present.

In this view, the barrel of the invention may be used with conventional stoppers which are generally used with conventional barrels free of external by-pass. Indeed, conventional stoppers are usually provided with three outer annular ribs forming a leak tight seal with the inner surface of a barrel. The first stopper may comprise a closed proximal end while the second stopper may comprise a proximal cavity enabling the connection with a plunger rod. Such embodiments may enable reducing the dead volume of the component located in the proximal chamber.

In embodiments, at least one of the first and second stoppers has a longitudinal length equal or smaller than the predetermined distance D. For example, the first stopper, i.e. the stopper which is located closest to the distal end of the barrel, has a longitudinal length equal or smaller than the predetermined distance D. As will appear clearly from the description below, a consequence of the presence of the longitudinal rib as described above in the barrel of the invention is the creation of a deformation of the outer annular ribs of the stoppers when these outer annular ribs are located in the transitional chamber. When a stopper has a longitudinal length equal or smaller than the predetermined distance D, which corresponds to the length of the transitional chamber, this means that all the outer annular ribs of this stopper, whatever their number, are allowed to be located in the transitional chamber simultaneously. As a consequence, when such a stopper is entirely located within the transitional chamber, all its outer annular ribs are deformed and the leak path thereby created extends from the proximal end of the stopper to its distal end. As will appear in the description below, the creation of such a leak path will allow the second component to transition from the proximal chamber either to the transitional chamber in order to be directly delivered, or to the distal chamber when present in order to reconstitute the liquid drug to be delivered.

In embodiments, each of the first and second stoppers has a longitudinal length equal or smaller than said predetermined distance D. For example, the first and the second stoppers are substantially identical. This allows simplifying the filling step of an injection device constructed from an assembly of the invention.

In embodiments, the first and second stoppers are dimensioned so that, in a contacted configuration of the stoppers, in which the second stopper is in contact with the first stopper and the second stopper is located proximally from the first stopper, the distance between a most distal outer annular rib of the first stopper and a most proximal outer annular rib of the second stopper is greater than the predetermined distance D. In other words, in such a configuration of the stoppers, at least one outer rib of the stoppers is in contact with the inner surface of the barrel out of the transitional chamber. As will appear from the detailed description below, this allows for example maintaining the tightness of the distal chamber, in a configuration where the first and the second stoppers are both at least partly located within the transitional chamber. With such an embodiment, the situation where both stoppers would be entirely located within the transitional chamber, and where by consequence a leak path would be present between the distal chamber and the proximal chamber with no leak tight seal of the proximal end of the barrel, cannot occur. Such an embodiment therefore ensures that, once reconstituted, the liquid drug to be delivered cannot leak out of the barrel via the proximal end of the barrel.

In embodiments, the first and second stoppers are further shaped and dimensioned so that, in their contacted configuration, at least two outer annular ribs of the plurality of outer annular ribs present on both first and second stoppers sealingly contact the inner surface of said tubular wall, regardless from the location of said stoppers in their contacted configuration within the interior of said barrel. Such an embodiment allows ensuring that no liquid drug of the distal chamber may leak backwards in the proximal direction, whatever the position of the stoppers.

As seen above, an injection device may be constructed with the assembly of the invention, where a first component is stored within the transitional chamber, or within the distal chamber when present, the first stopper is located at a distal end of the proximal chamber, the second stopper is located at a proximal end of the proximal chamber, and a second component is stored between the first stopper and the second stopper, in the proximal chamber. In such a configuration, the first stopper is located proximally from the transitional chamber, and all its outer annular ribs sealingly contact the inner wall of the proximal chamber. The second stopper is also entirely located within the proximal chamber and all its outer annular ribs sealingly contact the inner wall of the proximal chamber.

The first component may be in a liquid form, or in a dry form. Usually, when the first component is in a dry form, the barrel will comprise three chambers, and the first and second components will need to be mixed before being delivered. For instance, the first component may be a lyophilised drug and the second component may be a diluent of the lyophilised drug, so that the liquid drug to be delivered may be reconstituted by mixing the lyophilised drug and the diluent. Alternatively, both first and second components may be in the liquid form. In such a case, the barrel may comprise three chambers and the drug to be delivered may be a liquid intended to be reconstituted by mixing the first and the second component together. Alternatively, the barrel may comprise only two chambers, and each component may be delivered on its own, sequentially, one after the other.

The present application also describes a method for reconstituting a liquid drug to be delivered from an injection device having three chambers as described above, where the first component is stored in the distal chamber in a before use configuration. Such a method comprises the following steps :
- A plunger rod is assembled onto the second stopper,
- The user applies a distal pressure onto the plunger rod : this causes the movement of both stoppers in the distal direction, with the second component moving distally together with the stoppers,
- When the first stopper reaches the transitional chamber, the user continues applying a distal pressure onto the plunger rod; the gliding force increases as the longitudinal rib of the transitional chamber deforms the outer annular ribs of the first stopper;
- The user keeps pushing the plunger rod in the distal direction until all the outer annular ribs of the first stopper are located within the transitional chamber: as all the outer ribs of the first stopper have lost their tightness, a leak path is created, extending from a proximal end of the first stopper to its distal end and fluidly connecting the proximal chamber to the distal chamber;
- The user continues pushing distally on the plunger rod; as the second component, which is liquid, can now flow within the leak path, the gliding force needed to move the second stopper is lower than the gliding force needed to move the first stopper; as a consequence, the distal pressure exerted on the plunger rod by the user causes the second stopper to move distally, while the first stopper remains still in the transitional chamber; during this operation, all the second component transitions from the proximal chamber to the distal chamber via the leak path created at the level of the first stopper remaining still in the transitional chamber;
- When the second stopper comes in abutment against the first stopper, there is no second component left to be transitioned from the proximal chamber to the distal chamber; the user stops applying a distal pressure onto the plunger rod and he shakes the injection device to mix together the first and the second component and to reconstitute the liquid drug to be delivered; during this operation, since the second stopper is still in the proximal chamber, all its outer annular ribs sealingly contact the inner wall of said proximal chamber; as a result, the second stopper constitutes a leak tight seal preventing the reconstituted liquid drug to leak out in the proximal direction;
- Once the liquid drug is reconstituted, the user applies again a distal pressure onto the plunger rod, thereby moving both stoppers together; the gliding force for moving both stoppers increases; the stoppers are in their contacted configuration as described above; as the user continues pushing distally on the plunger rod, the liquid drug is expelled through the passageway at the distal end of the barrel, and then through the cannula, and the injection is completed.

The invention and the advantages arising therefrom will clearly emerge from the detailed description that is given below with reference to the appended drawings as follows :
- Figure 1A is a longitudinal section view of a first embodiment of a barrel of the invention,
- Figure 1B is a cross section view taken along plane I-I of Figure 1A,
- Figure 1C is a cross section view of the barrel of Figure 1 B with a stopper located in the transitional chamber,
- Figure 2A is a cross section view of a second embodiment of a barrel of the invention,
- Figure 2B is a cross section view of the barrel of Figure 2A with a stopper located in the transitional chamber,
- Figure 3A is a cross section view of a third embodiment of a barrel of the invention,
- Figure 3B is a cross section view of the barrel of Figure 3A with a stopper located in the transitional chamber;
- Figure 4 is a cross section view of a fourth embodiment of the barrel of the invention, with a stopper in the transitional chamber,
- Figure 5 is a cross section view of a fifth embodiment of the barrel of the invention, with a stopper in the transitional chamber,
- Figure 6 is a cross section view of a sixth embodiment of the barrel of the invention, with a stopper in the transitional chamber,
- Figures 7A and 7B are respectively a partial longitudinal section view and a partial top view of a seventh embodiment of the barrel of the invention,
- Figures 8A and 8B are respectively a partial longitudinal section view and a partial top view of an eighth embodiment of the barrel of the invention,
- Figures 9A and 9B are respectively a partial longitudinal section view and a partial top view of a ninth embodiment of the barrel of the invention,
- Figures 10A and 10B are respectively a partial longitudinal section view and a partial top view of a tenth embodiment of the barrel of the invention,
- Figure 11A is a longitudinal section view of an injection device comprising the barrel of Figure 1A, in which a first component is stored in the distal chamber, a second component is stored in the proximal chamber, a first stopper is located at a distal end of the proximal chamber and a second stopper is located at the proximal end of the proximal chamber, where said injection device is in a before use position,
- Figure 11B is cross section view taken along plane II-II of Figure 11A,
- Figure 12A is a longitudinal section view of the injection device of Figure 11A at the beginning of use of the injection device, when the first stopper enters the transitional chamber,
- Figure 12B is a cross section view taken along plane III-III of Figure 12A,
- Figure 13A is a longitudinal section view of the injection device of Figure 11A when the first stopper is entirely located within the transitional chamber,
- Figure 13B is a cross section view taken along plane IV-IV of Figure 13A,
- Figure 14A is a longitudinal section view of the injection device of Figure 11A when the first stopper is entirely located within the transitional chamber and the second component transitions from the proximal chamber to the distal chamber,
- Figure 14B is a cross section view taken along plane V-V of Figure 14A,
- Figure 15A is a longitudinal section view of the injection device of Figure 11A once all the second component has left the proximal chamber and after mixing of the two components in the distal chamber,
- Figure 15B is a cross section view taken along plane VI-VI of Figure 15A,
- Figure 16A is a longitudinal section view of the injection device of Figure 11A when the second stopper enters the transitional chamber,
- Figure 16B is a cross section view taken along plane VII-VII of Figure 16A,
- Figure 16C is a cross section view taken along plane VIII-VIII of Figure 16A,
- Figure 16D is a cross section view taken along plane IX-IX of Figure 16A,
- Figure 17A is a longitudinal section view of the injection device of Figure 11A during the injection step,
- Figure 17B is a cross section view taken along plane X-X of Figure 17A,
- Figure 17C is a cross section view taken along plane XI-XI of Figure 17A,
- Figure 17D is a cross section view taken along plane XII-XII of Figure 17A,
- Figure 17E is a cross section view taken along plane XIII-XIII of Figure 17A,
- Figure 18A is a longitudinal section view of the injection device of Figure 11A at the end of the injection step,
- Figure 18B is a cross section view taken along plane XIV-XIV of Figure 18A,
- Figure 19A is a longitudinal section view of an injection device comprising another embodiment of the barrel of the invention in which the barrel comprises only two chambers, in which a first component is stored in the transitional chamber, a second component is stored in the proximal chamber, a first stopper is located at a distal end of the proximal chamber and a second stopper is located at the proximal end of the proximal chamber, where said injection device is in a before use position,
- Figure 19B is a cross section view taken along plane XV-XV of Figure 19A,
- Figure 20A is a longitudinal section view of the injection device of Figure 19A at the beginning of use of the injection device, when the first stopper enters the transitional chamber,
- Figure 20B is a cross section view taken along plane XVI-XVI of Figure 20A,
- Figure 21A is a longitudinal section view of the injection device of Figure 19A when the first stopper is entirely located within the transitional chamber and the second component transitions from the proximal chamber to the transitional chamber,
- Figure 21B is a cross section view taken along plane XVII-XVII of Figure 21A,
- Figure 22A is a longitudinal section view of the injection device of Figure 19A when the second component enters the distal end of the proximal chamber,
- Figure 22B is a cross section view taken along plane XVIII-XVIII of Figure 22A,
- Figure 23A is a longitudinal section view of the injection device of Figure 19A at the end of the injection step,
- Figure 23B is a cross section view taken along plane XIX-XIX of Figure 23B.

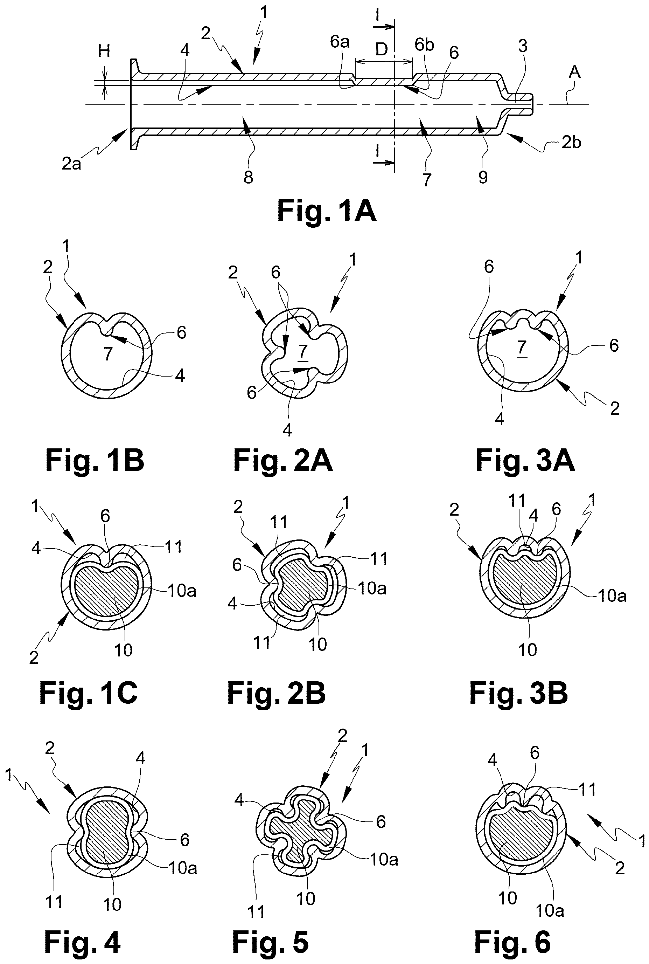

With reference to Figures 1A-1C will now be described a first embodiment of the barrel 1 of the invention, in which the barrel 1 comprises three chambers (7, 8, 9). The barrel 1 is formed of a tubular wall 2 having a longitudinal axis A and a proximal end 2a and a distal end 2b. The proximal end 2a is open. In the example shown, the proximal end 2a of the tubular wall 2 forms an outer flange. Anyway, in other embodiments not shown, the outer flange could be a separate part removably attachable to the proximal end of the barrel 1. In the example shown, the distal end 2b is tapered in the distal direction and defines a passageway 3. The passageway 3 is intended to be connected to a needle cannula or a needle hub (not shown) for delivery of a product intended to be stored within the barrel 1.

The tubular wall 2 has an inner surface 4 defining an interior 5 of the barrel 1. The inner surface 4 is provided with a longitudinal rib 6 extending longitudinally on a predetermined distance D.

As shown in Figure 1A, the longitudinal rib 6 has a proximal end 6a and a distal end 6b and is dimensioned and positioned to divide the interior 5 of the barrel 1 into three chambers, a transitional chamber 7, extending on the predetermined distance D between the proximal end 6a and the distal end 6b of the rib 6, a proximal chamber 8 located proximally to the transitional chamber 7, and a distal chamber 9 located distally to the transitional chamber 7. The transitional chamber 7 therefore separates the distal chamber 9, the distal end of which communicates with the passageway 3, from the proximal chamber 8, the proximal end of which is common with the proximal end 2a of the tubular wall 2.

As shown in Figure 1A, the inner diameter of the proximal chamber 8 and the inner diameter of the distal chamber 9 are identical and they are constant on the whole length of each of the proximal chamber 8 and the distal chamber 9. The longitudinal rib 6 is directed radially inwardly, towards the central longitudinal axis A of the tubular wall 2, and has a radial inward depth H. As a consequence, the actual inner volume of the transitional chamber 7 is smaller than what the inner volume of the transitional chamber 7 would be if said transitional chamber 7 did not comprise the longitudinal rib 6.

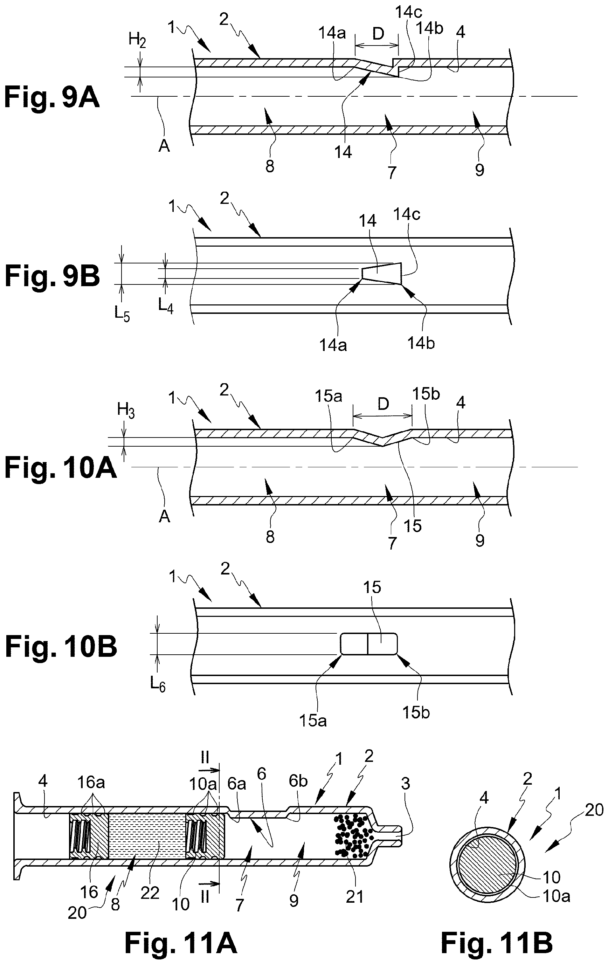

With reference to Figure 1C, the barrel 1 is shown with a stopper, for example a first stopper 10, located in the transitional chamber 7. With reference to Figure 11A, the first stopper 10 has a globally cylindrical shape and is provided with three outer annular ribs 10a. The annular ribs 10a are dimensioned to sealingly contact the inner surface 4 of the tubular wall 2, when the first stopper 10 is either in the proximal chamber 8 or in the distal chamber 9. As a consequence, when an annular outer rib 10a is located in the transitional chamber 7, as shown in Figure 1C, the annular outer rib 10a is deformed by the longitudinal rib 6 and it is prevented from sealingly contact the inner surface 4 of the tubular wall 2 on its entire circumference. The result is that a part of the annular outer rib 10a does not contact the inner surface 4 of the tubular wall 2 and a leak path 11 is created, as shown in Figure 1C. As will appear in the description below, the creation of such a leak path 11 will allow the reconstitution of a liquid drug 23 from a first component 21 stored in the proximal chamber 8 and a second component 22 stored in the distal chamber 9 (see Figures 11A-18B).

In the embodiment shown in Figures 1A-1C, only one longitudinal rib 6 is present on the inner surface 4 of the tubular wall 2.

With reference to Figures 2A-6, are shown embodiments of the barrel 1 where a plurality of longitudinal ribs 6 are present on the inner surface 4 of the tubular wall 2. The longitudinal ribs 6 may be regularly distributed along a circumference of the tubular wall 2 : this is the case in embodiments of Figures 2A and 2B, where three longitudinal ribs 6 are present, of Figure 4, where two diametrically opposed longitudinal ribs 6 are present, and of Figure 5, where four longitudinal ribs 6 are present. Alternatively, the longitudinal ribs 6 may be confined within a restricted arc of the circumference of the tubular wall 2, as shown in the embodiment of Figures 3A and 3B, where two longitudinal ribs 6 are present, or in the embodiment of Figure 6, where three longitudinal ribs 6 are present. In all these embodiments, the longitudinal ribs 6 will deform the outer annular rib 10a of the first stopper 10 when this first stopper 10 is located in the transitional chamber 7 and a leak path 11 will be created in the same manner as described above (see Figures 2B, 3B and 4-6).

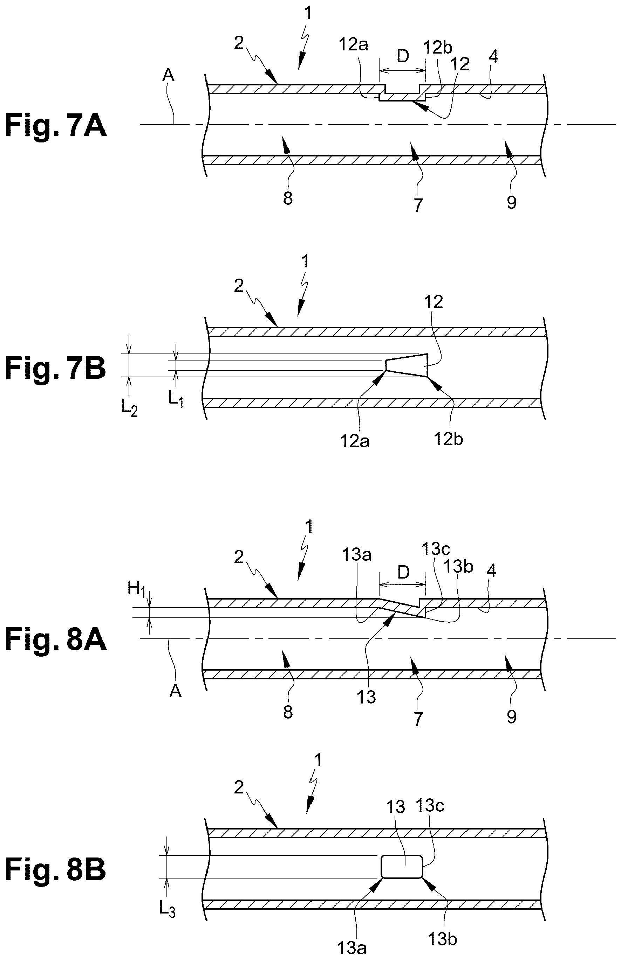

With reference to Figures 7A and 7B is shown another embodiment of the barrel 1 of the invention, in which the circumferential length of the longitudinal rib 12 varies from a proximal end 12a of the longitudinal rib 12 to a distal end 12b of the longitudinal rib 12, and where the radial inward height of the longitudinal rib 12 remains constant on the distance D. In the example shown, the circumferential length L1 of the longitudinal rib 12 at its proximal end 12a is smaller than the circumferential length L2 of the longitudinal rib 12 at its distal end 12b. Because of the shape of this longitudinal rib 12, the width of the leak path created when a stopper (not shown) is present in the transitional chamber 7 progressively increases in the distal direction : this allows smoothening the increase of the gliding force needed to move the stopper distally in the transitional chamber 7.

With reference to Figures 8A and 8B is shown an alternative embodiment of the barrel 1 of the invention, in which the circumferential length L3 of the longitudinal rib 13 remains constant on the distance D, but where the radial inward height varies from a proximal end 13a of the longitudinal rib 13 to a distal end 13b of the longitudinal rib 13. More precisely, the radial inward height progressively increases from 0 at the proximal end 13a to a value of H1 at the distal end 13b. For example, the value of H1 is chosen so that a step 13c is created in the inner surface 4 of the tubular wall 2 of the barrel at the distal end 13b of the longitudinal rib 13. The presence of such a step 13c generates a clear drop in the gliding force needed to move a stopper (not shown) distally in the transitional chamber 7. This clear drop is a tactile indication for the user that the stopper leaves the transitional chamber. The step 13c also constitutes a visual indication for the user that the stopper exits the transitional chamber 7 and enters the distal chamber 9.

With reference to Figures 9A and 9B is shown another embodiment of the barrel 1 of the invention, in which the circumferential length L4 at the proximal end 14a of the longitudinal rib 14 is smaller than the circumferential length L5 at the distal end 14b of the longitudinal rib 14, and the radial inward height of the longitudinal rib gradually increases from 0 at the proximal end 14a of the longitudinal rib 14 to a significant value H2 to form a step 14c at the distal end 14b of the longitudinal rib 14. Such an embodiment combines the benefits described above for the embodiments of Figures 7A-8B.

With reference to Figures 10A and 10B, is shown another embodiment of the barrel 1 of the invention, in which the radial inward height of the longitudinal rib 15 gradually increases from 0 to a maximum value H3 and then decreases back to 0 along the distance D. In the example shown, the circumferential length L6 remains constant from the proximal end 15a of the longitudinal rib 15 to its distal end 15b. With such an embodiment, the effort the user needs to exert to move a stopper (not shown) distally in the transitional chamber 7 may be varied : the gliding force needed to move the stopper smoothly increases up to the point where the height of the longitudinal rib 15 is the highest and then smoothly decreases until the distal end 15b of the longitudinal rib 15.

The barrel 1 may be made of plastic or of glass material. It is intended to be used in combination with two stoppers, a first stopper 10 and a second stopper 16, as will appear from Figures 11A-18B. The first and the second stoppers (10, 16) are shaped and dimensioned to be slidable within the interior 5 of the barrel 1. In the example shown, the first and the second stoppers (10, 16) are both provided with three outer annular ribs (10a, 16a) which are dimensioned to sealingly contact the inner surface 4 of the tubular wall 2 when the stoppers (10, 16) are located either within the proximal chamber 8 or within the distal chamber 9.

In embodiments not shown, the first stopper could be different from the second stopper: for example, the two stoppers could have different lengths or they could have a different number of outer annular ribs.

With reference to Figure 11A is shown an injection device 20 made from the barrel 1 of Figure 1 in a before use or storage position. For clarity's sake, neither the needle cannula nor the plunger rod are represented in Figures 11A-18B. A first component 21 in a dry form is stored in the distal chamber 9 and a second component 22 in a liquid form is stored in the proximal chamber 8. The first stopper 10 is located at a distal end of the proximal chamber 8 and the second stopper 16 is located at a proximal end of the proximal chamber 8.

The method for reconstituting a liquid drug by mixing the first component 21 and the second component 22 thanks to the injection device 20 and then for expelling this liquid drug during an injection step will now be described with reference to Figures 11A-18B. All the reconstitution steps described below must be done with the passageway 3 of the injection device 20 in the upward direction in order to enable the air bubbles possibly present in the chambers (7, 8, 9) to be expelled before any liquid drug is.

As appears from Figures 11A and 11B, in the storage configuration of the injection device 20, the first stopper 10 and the second stopper 16 are both fully located within the proximal chamber 8, and all their respective outer annular ribs (10a, 16a) are in contact with the inner surface 4 of the tubular wall 2 (see Figure 11B) thereby constituting leak tight seals in the distal direction and in the proximal direction with respect to the second component 22 which may not leak out of the proximal chamber 8.

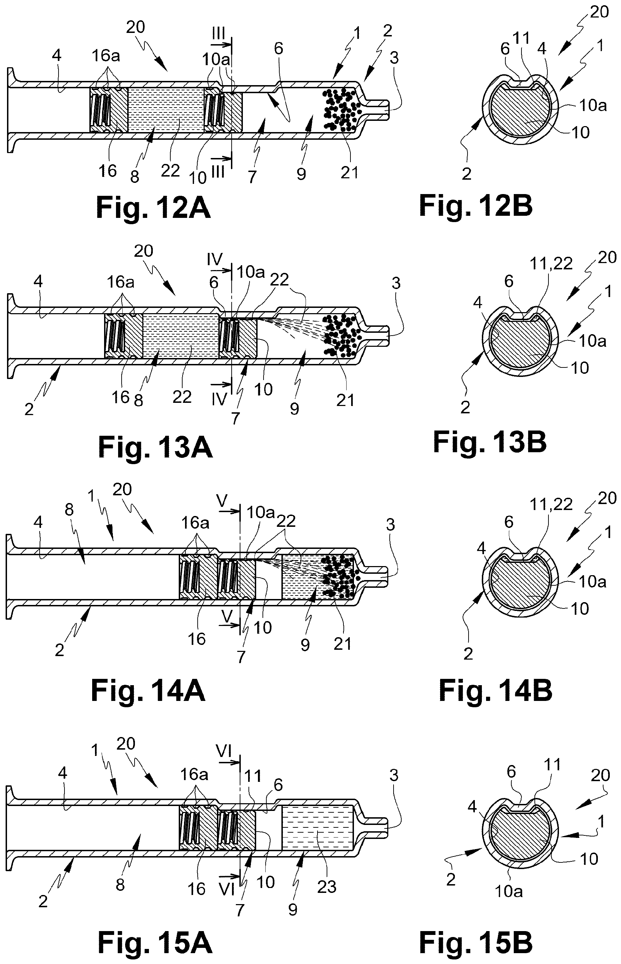

When a user wishes to begin the reconstitution of the liquid drug 23 to be injected (see Figure 15A), he applies a distal pressure onto a plunger rod connected to the proximal end of the second stopper 16. As a result, the second stopper 16, the second component 22 and the first stopper 10 all move together in the distal direction. When the first stopper 10 reaches the transitional chamber 7, as shown in Figures 12A and 12B, the gliding force needed to move the stopper 10 distally increases, as a first outer annular rib 10a of the first stopper 10 comes in contact with the longitudinal rib 6 and is deformed by this longitudinal rib 6. With reference to Figure 12B, the deformation of the first outer annular rib 10a of the first stopper 10 creates a leak path 11 at the level of this first outer annular rib 10a. Nevertheless, at this stage, since some more proximal outer annular ribs 10a of the first stopper 10 are still located within the proximal chamber 8, the leak path 11 does not extend on the whole length of the first stopper 10 and this first stopper 10 has not lost its tightness. The second component 22 is not allowed to transition from the proximal chamber 8 to the distal chamber 9.

The user then continues applying a distal force on the second stopper 16 until all the outer annular ribs 10a of the first stopper 10 are located within the transitional chamber 7, as shown in Figure 13A. As appears from this Figure, the longitudinal length of the first stopper 10 is smaller than the distance D (see Figure 1A) on which the longitudinal rib 6 extends. As a result, the three outer annular ribs 10a of the first stopper 10 are deformed by the longitudinal rib 6 and the leak path 11 thereby created extends on the whole longitudinal length of the first stopper 10 and fluidly connects the proximal chamber 8 to the distal chamber 9. As shown in Figures 13A and 13B, the second component 22, which is in a liquid form, is therefore allowed to flow through the leak path 11 and to transition from the proximal chamber 8 to the distal chamber 9.

The user then continues applying a distal force on the second stopper 16. As the second component 22, which is liquid, can now flow within the leak path 11, the gliding force needed to move the second stopper 16 is lower than the gliding force needed to move the first stopper 10. The first stopper 10 therefore remains immobile while the distal movement of the second stopper 16 causes the whole component 22 to transition from the proximal chamber 8 to the distal chamber 9 via the leak path 11, as shown in Figures 14A and 14B. The second stopper 16 comes in abutment against the first stopper 10 and only a tiny portion of the second component 22 may remain stuck in the leak path 11, the majority of the second component 22 having reached the distal chamber 9.

At this stage, the user stops applying a distal force on the second stopper 16 and he shakes the injection device 20 in order to mix the first component 21 and the second component 22 together. During this shaking step, there is no risk that some of the first or second components (21, 22) escapes through the proximal end of the distal chamber 9. Indeed, as appears from Figure 15A, the second stopper 16 is still fully located in the proximal chamber 8 and its three outer annular ribs 16a sealingly contact the inner surface 4 of the tubular wall 2, thereby realising a leak tight seal. At the end of the shaking step, the liquid drug 23 to be injected is reconstituted and is fully located in the distal chamber 9, as shown in Figure 15A.

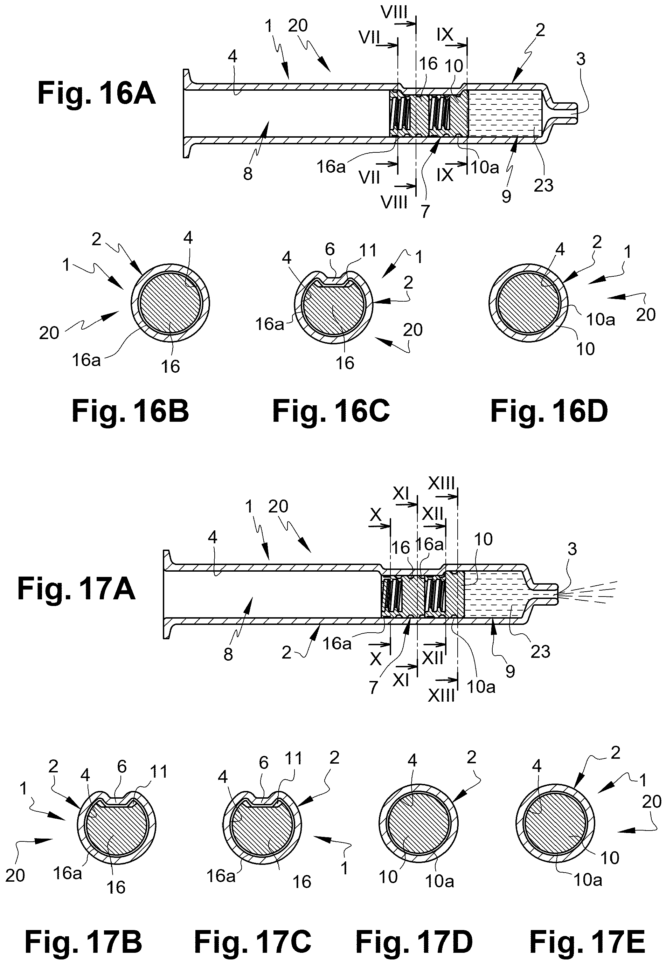

The user then starts again applying a distal force on the second stopper 16 so as to move distally both stoppers (10, 16) : as shown in Figure 16A, the first stopper 10 and the second stopper 16 are in a contacted configuration, where the second stopper 16 is in contact with the first stopper 10. The distal region of the second stopper 16 starts entering the transitional chamber 7 while the distal region of the first stopper 10 starts exiting said transitional chamber 7. As shown in Figures 16A, 16B and 16D, the distance between the most distal outer annular rib 10a of the first stopper 10 and the most proximal outer annular rib 16a of the second stopper 16 is greater than the distance D (see Figure 1A) on which the longitudinal rib 6 extends. As a result this most distal outer annular rib 10a of the first stopper 10 and this most proximal outer annular rib 16a of the second stopper 16 both sealingly contact the inner surface 4 of the tubular wall 2 (see Figures 16B and 16D), thereby realising a leak tight seal. Therefore, although some of the other outer annular ribs (10a, 16a) of the first stopper 10 and of the second stopper 16, which are in contact with the longitudinal rib 6 may be deformed and may have lost their tightness, as shown in Figure 16C, the liquid drug 23 is safely confined within the distal chamber 9 and it is not allowed to leak back in the proximal direction.

To proceed with the injection into a patient through the needle cannula or the needle hub (not shown), the user keeps applying a distal force onto the second stopper 16. Both stoppers (10, 16) are moved distally and the liquid drug 23 is expelled through the passageway 3 as shown in Figure 17A. With reference to figures 17B-17E, it can be observed that during the injection step, at least two annular outer ribs 10a of the first stopper 10 are always in sealing contact with the inner surface 4 of the tubular wall 2. This allows ensuring that the liquid drug 23 may not leak out from the distal chamber 9 in the proximal direction.

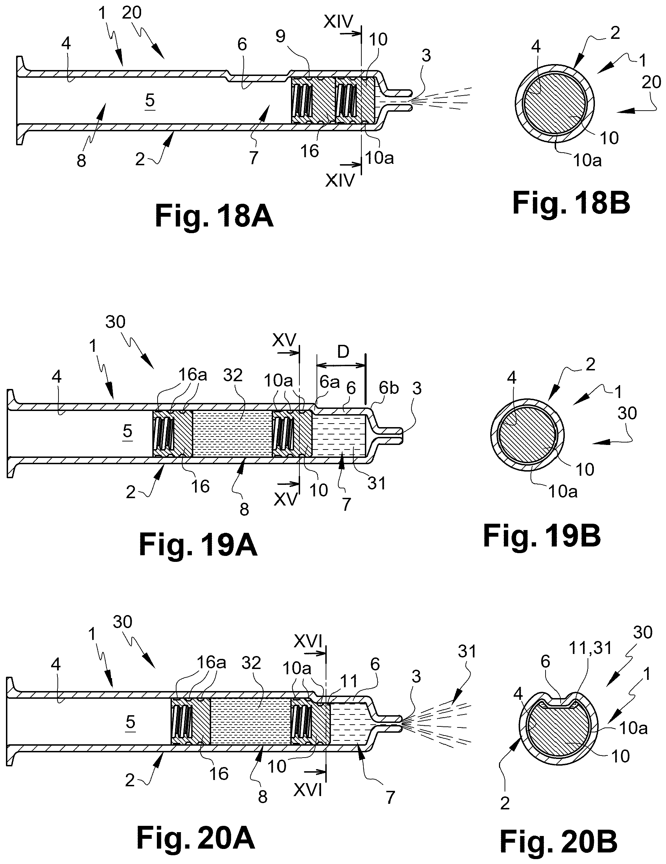

With reference to Figures 18A and 18B, the injection device 20 is shown at the end of injection, when the first stopper 10 comes in abutment against the passageway 3. All the liquid drug 23 has been injected. Both stoppers (10, 16) are fully located within the distal chamber 9. The first stopper 10 and the second stopper 16 are in their contacted configuration.

With reference to Figures 19A-23B is shown an injection device 30 made from a barrel 1 of the invention in which the longitudinal rib 6 is dimensioned and positioned to divide the interior 5 of the barrel 1 into two chambers only, the transitional chamber 7 and the proximal chamber 8. The reference signs used for Figures 11A-18B and designating the same elements are maintained in Figures 19A-23B. The injection device 30 allows injecting two liquids components which need to be stored separately before injection, and then delivered sequentially during the injection.

As appears from Figures 19A-23B, the longitudinal rib 6 extends on a distance D up to the distal end of the barrel 1 and no distal chamber is present.

In Figure 19A, the injection device 30 is in its before use or storage configuration : a first liquid component 31 is stored in the transitional chamber 7 and a second liquid component 32 is stored in the proximal chamber 8. The first stopper 10 is located at a distal end of the proximal chamber 8 and the second stopper 16 is located at a proximal end of the proximal chamber 8. The first stopper 10 and the second stopper 16 are both fully located within the proximal chamber 8, and all their respective outer annular ribs (10a, 16a) are in contact with the inner surface 4 of the tubular wall 2 (see Figure 19B) thereby constituting leak tight seals in the distal direction and in the proximal direction with respect to the second component 32 which may not leak out of the proximal chamber 8.

In order to start the injection of the first component 31, the user applies a distal pressure onto a plunger rod (not shown) connected to the proximal end of the second stopper 16. The second stopper 16, the second component 32 and the first stopper 10 all move together in the distal direction. Under the pressure of the first stopper 10, the first component 31 is expelled and exits the barrel 1 through the passageway 3, as shown in Figure 20A.

Simultaneously, the first stopper 10 reaches the transitional chamber 7, as shown in Figures 20A and 20B, the gliding force needed to move the stopper 10 distally increases, as a first outer annular rib 10a of the first stopper 10 comes in contact with the longitudinal rib 6 and is deformed by this longitudinal rib 6. With reference to Figure 20B, the deformation of the first outer annular rib 10a of the first stopper 10 creates a leak path 11 at the level of this first outer annular rib 10a.

Nevertheless, at this stage, since some more proximal outer annular ribs 10a of the first stopper 10 are still located within the proximal chamber 8, the leak path 11 does not extend on the whole length of the first stopper 10 and this first stopper 10 has not lost its tightness. The second component 32 is therefore not yet allowed to transition from the proximal chamber 8 to the transitional chamber 7.

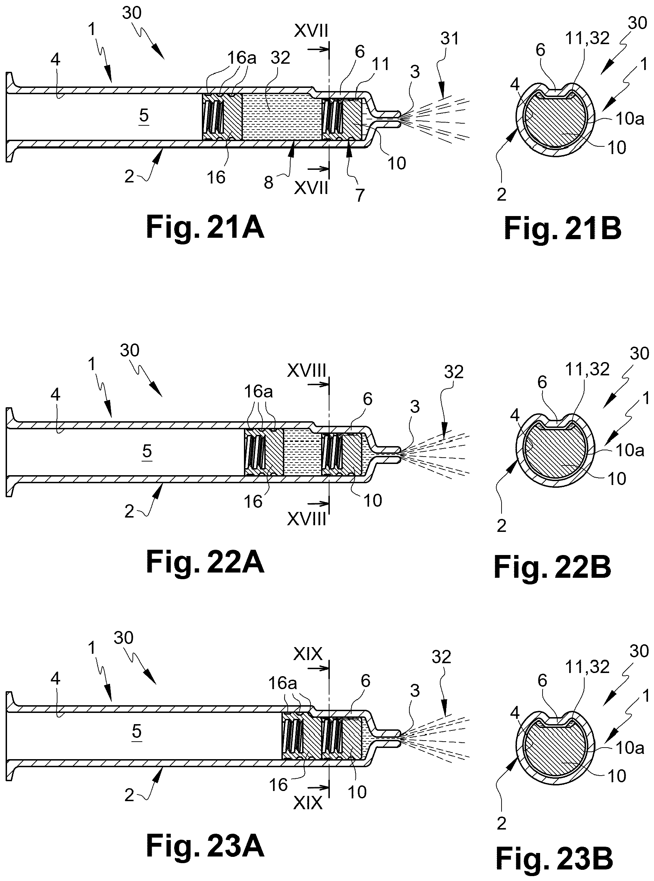

The user then continues applying a distal force on the second stopper 16 until the distal end of the first stopper 10 comes in abutment against the distal end of the barrel 1. At this stage, almost all the first component 31 has been expelled and all the outer annular ribs 10a of the first stopper 10 are located within the transitional chamber 7, as shown in Figure 21A. This is possible because the longitudinal length of the first stopper 10 is smaller than the distance D (see Figure 19A). As a result, all the outer annular ribs 10a of the first stopper 10 have lost their tightness and the leak path 11 now extends on the whole length of the first stopper 10 and fluidly connects the proximal chamber 8 to the transitional chamber 7. The second liquid component 32 is allowed to flow from the proximal chamber 8 to the transitional chamber 7, as shown in Figure 21B.

The user continues applying a distal force on the second stopper 16. As the first stopper 10 can no longer move distally, the second component 32 flows through the leak path 11 up to the passageway 3 and is expelled from the barrel 1, as shown in Figures 22A and 22B.

As the user keeps pushing distally on the second stopper 16, the injection of all the second component 32 is completed, as shown in Figures 23A and 23B.

The barrel 1 of Figures 19A-23B therefore allows injecting sequentially, i.e. one after the other, two liquid components stored separately before the injection.

As seen above, the barrel 1 of the invention is easy to manufacture and easy to manipulate during the various operations it is intended to undergo, such as filling, finishing, conveying, packaging, storing, etc... In combination with stoppers, which may be conventional stoppers, the barrel 1 of the invention allows storing separately two components intended to be either delivered sequentially one after the other, or intended to be mixed to reconstitute a liquid drug just before the injection.

## Claims

1. A barrel (1) for an injection device (20), said barrel (1) being formed of a tubular wall (2) with an open proximal end (2a) and a distal end (2b) defining a passageway (3) for a product (21, 22, 23) to be stored in said barrel (1), said tubular wall (2) having an inner surface (4) defining an interior (5) of said barrel (1), wherein said inner surface (4) is provided with at least one radially inwardly directed longitudinal rib (6; 12; 13; 14; 15) extending longitudinally on a predetermined distance D, said longitudinal rib (6; 12; 13; 14; 15) being dimensioned and positioned to divide the interior (5) of said barrel (1) into at least two chambers, a transitional chamber (7), extending on said predetermined distance D, and a proximal chamber (8) located proximally to said transitional chamber (7).

2. The barrel (1) of claim 1, wherein said longitudinal rib (6; 12; 13; 14; 15) is dimensioned and positioned to divide the interior (5) of said barrel (1) into three chambers which are said transitional chamber (7), said proximal chamber (8), and a distal chamber (9) which is located distally to said transitional chamber (7).

3. The barrel (1) of claim 1 or 2, comprising a plurality of said longitudinal ribs (6) distributed along a circumference of said tubular wall (2).

4. The barrel (1) of claim 3, wherein the longitudinal ribs (6) are regularly distributed along said circumference of said tubular wall (2).

5. The barrel (1) of any one of claims 1-4, wherein the circumferential length (L1, L2; L4, L5) of the longitudinal rib(s) (12; 14) varies from a proximal end (12a; 14a) of the longitudinal rib(s) (12; 14) to a distal end (12b; 14b) of the longitudinal rib(s) (12; 14).

6. The barrel (1) of claim 5, wherein the circumferential length of a longitudinal rib (12; 14) is smaller at the proximal end (12a; 14a) of the longitudinal rib (12; 14) than at its distal end (12b; 14b).

7. The barrel (1) of any one of claims 1-6, wherein the radial inward height (H1; H2, H3) of the longitudinal rib(s) (13; 14; 15) varies from a proximal end (13a; 14a; 15a) of the longitudinal rib(s) (13; 14; 15) to a distal end (13b; 14b; 15b) of the longitudinal rib(s) (13; 14; 15).

8. The barrel (1) of claim 7, wherein the radial inward height of a longitudinal rib (13; 14) gradually increases from 0 at the proximal end (13a; 14a) of the longitudinal rib (13; 14) to a significant value (H1; H2) at the distal end (13b; 14b) of the longitudinal rib (13; 14), said significant value being chosen to create a step (13c; 14c) in the inner surface (4) of the tubular wall (2).

9. The barrel (1) of claim 7, wherein the radial inward height of a longitudinal rib (15) gradually increases from 0 to a maximum value (H3) and then decreases back to 0 along the length (D) of the longitudinal rib (15).

10. An assembly comprising :
- At least one barrel (1) according to any one of claims 1-9, and
- At least one first stopper (10) and one second stopper (16), each of said first and second stoppers (10, 16) being shaped and dimensioned to be slidable within the interior (5) of the barrel (1), each of said first and second stoppers (10, 16) being provided with at least one outer annular rib (10a, 16a) dimensioned to sealingly contact the inner surface (4) of said tubular wall (2).

11. Assembly according to claim 10, wherein each stopper (10, 16) is provided with a plurality, for example three, outer annular ribs (10a, 16a) dimensioned to sealingly contact the inner surface (4) of said tubular wall (2).

12. Assembly according to claim 10 or 11, wherein at least one of said first and second stoppers (10, 16) has a longitudinal length equal or smaller than said predetermined distance D.

13. Assembly according to any one of claims 10-12, wherein said first and second stoppers (10, 16) are dimensioned so that, in a contacted configuration of the stoppers, in which said second stopper (16) is in contact with said first stopper (10) and said second stopper (16) is located proximally from said first stopper (10), the distance between a most distal outer annular rib (10a) of said first stopper (10) and a most proximal outer annular rib (16a) of said second stopper (16) is greater than said predetermined distance D.

14. Assembly according to claim 13, wherein said first and second stoppers (10, 16) are further shaped and dimensioned so that, in their contacted configuration, at least two outer annular ribs (10, 16a) of the plurality of outer annular ribs (10a, 16a) present on both first and second stoppers (10, 16) sealingly contact the inner surface (4) of said tubular wall (2), regardless from the location of said stoppers (10, 16) in their contacted configuration within the interior (5) of said barrel (2).

15. Injection device (20) comprising an assembly according to any one of claims 10-14, wherein :
- i) a first component (21) is stored within said transitional chamber (7) when said barrel (1) comprises a transitional chamber (7) and a proximal chamber (8) only, or
- ii) a first component (21) is stored within said distal chamber (9) when said barrel (1) comprises a transitional chamber (7), a proximal chamber (8) and a distal chamber (9),
and said first stopper (10) is located at a distal end of said proximal chamber (8), said second stopper (16) is located at a proximal end of said proximal chamber (8), and a second component (22) is stored between said first stopper (10) and said second stopper 16), in the proximal chamber (8).
